(19) Europäisches Patentamt / European Patent Office / Office européen des brevets

(11) EP 3 856 749 B1

(12) EUROPEAN PATENT SPECIFICATION

(45) Date of publication and mention of the grant of the patent:
**01.05.2024 Bulletin 2024/18**

(21) Application number: **19836619.7**

(22) Date of filing: **11.07.2019**

(51) International Patent Classification (IPC):
***C07F 7/02*** (2006.01) ***A61P 35/00*** (2006.01)
***A61K 31/695*** (2006.01) ***A61K 41/00*** (2020.01)

(52) Cooperative Patent Classification (CPC):
**C07F 7/025; A61K 41/0071; A61P 35/00**

(86) International application number:
**PCT/TR2019/050566**

(87) International publication number:
**WO 2020/068015 (02.04.2020 Gazette 2020/14)**

(54) **WATER-SOLUBLE, NON-AGGREGATED SILICON PHTALGCYANINE COMPOUND HAVING IN VITRO ANTICANCER EFFECT AGAINST CANCER TYPES OF LUNG, LIVER, BREST AND MELANOMA**

WASSERLÖSLICHE NICHT-AGGREGIERTE PHTHALOCYANINVERBINDUNG MIT ANTIKREBSWIRKUNG GEGEN LUNGEN-, LEBER-, BRUSTKREBS UND MELANOME

COMPOSÉ DE PHTALOCYANINE DE SILICIUM NON-AGRÉGÉ SOLUBLE DANS L'EAU AYANT UN EFFET ANTICANCÉREUX IN VITRO CONTRE LES CANCERS DU POUMON, DU FOIE, DU SEIN ET LE MÉLANOME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.09.2018 TR 201814010**
**26.09.2018 TR 201814012**

(43) Date of publication of application:
**04.08.2021 Bulletin 2021/31**

(73) Proprietor: **Karadeniz Teknik Universitesi**
**61080 Trabzon (TR)**

(72) Inventors:
- **BIYIKLIOGLU, Zekeriya**
  **Kimya A.B.D Trabzon (TR)**
- **OZEL, Arzu**
  **Ortahisar/Trabzon (TR)**
- **BARUT, Burak**
  **Ortahisar/Trabzon (TR)**
- **BAS, Huseyin**
  **Ortahisar/Trabzon (TR)**

(74) Representative: **Sevinç, Cenk**
**Grup Ofis Marka Patent A.S.**
**Ataturk Bulvari 211/11**
**Kavaklidere**
**06680 Ankara (TR)**

(56) References cited:
**CN-A- 107 474 064**

- **OMEROGLU, IPEK; KAYA, ESRA NUR; GOKSEL, MELTEM; KUSSOVSKI, VESSELIN; MANTAREVA, VANYA; DURMUS, MAHMUT: "Axially substituted silicon(IV) phthalocyanine and its quaternized derivative as photosensitizers towards tumor cells and bacterial pathogens", BIOORGANIC & MEDICINAL CHEMISTRY (2017), 25(20), 5415-5422, vol. 25, no. 20, 1 August 2017 (2017-08-01), pages 5415-5422, XP002797482, ISSN: 0968-0896**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 856 749 B1

## Description

### Technical Field

The present invention relates to silicon phthalocyanine compounds and synthesis method of these compounds, which constitute an activity in cancer cells resistant to compounds having metal and whose effect spectrum is wider compared to metalliferous compounds and whose toxic effect is lower compared metalliferous compounds and whose side effects are reduced and which can be used as potential antitumor drug and which comprise anticancer and antioxidant effects and which have groups of (3,5-bis{3-[3-(diethylamino)phenoxy]propoxy}phenyl)methoxy or (3,5-bis{3-[3-(dimethylamino)phenoxy]propoxy}phenyl)methoxy.

### State of the Art

Tissues of healthy species, throughout their lifetime, are renewed by means of continuous cell division until their cells are dead. It is required to maintain the balance between cell death and cell division so as to sustain the life. Damaging to genetic materials causes the balance to fail and cells to reproduce and grow in an uncontrollable manner. Occurrence of a tissue with damaged DNA cannot be treated in general. In many cases, such tissues cause tumor or cancer to develop.

Treatment methods used mostly in cancer cases are surgery, radiotherapy and chemotherapy. Hormonal therapies, biological therapy methods and targeted treatments are used less frequently and said treatment methods are implemented alone or in combination. Treatment methods and medicines administered may vary according to type and stage of the tumor. Furthermore, response received from the treatment may vary according to metabolism of patients.

Currently, cancer is the second in Europe to cause death. Therefore, efficient treatment of the disease is of very important. However, cancer is a very heterogeneous and complexed disease. While Surgery and radiotherapy cures 40 percent of cancer patients, the remaining 60 percent of the patients loses their lives due to metastasis. Within that period, because of systematical nature of the disease, a systematical treatment like a chemotherapy is needed. Chemotherapy has a high curative effect for various types of cancers despite of a highly metastatic spreading feature. However, chemotherapy does not have a long-term curative properties in patients with advanced disease. Development of the chemotherapy constitutes a great importance so as to both extend curable tumor spectrum and to improve life quality of patients under treatment.

The leading one of chemotherapeutic treatments is those molecules targeting DNA. Intact DNA is one of the crucial conditions so that cells can fulfill their functions and grow. In normal cells, cell cycle controlling proteins is in charge of identifying DNA damages occurred and either stopping cell cycle according to size of the damage or activating pathways causing cell to kill itself. Particularly, DNA lesions occurring in the phase S of cell cycle cause replication fork to stop and double-chained DNA fragmentation to occur, which can be identified as the most toxic one among DNA damage types. Cell death occurs in case damaged DNA cannot be repaired. In cancer cells, pathways pertaining detecting or repairing DNA damages are weakened generally. Furthermore, cancer cells having a feature ignoring cell cycle controlling points reach up to higher proliferation rates. This causes that cancer cells are more sensitive to DNA damages. Nowadays, cancer medicines targeting DNA are considered as the most effective and used treatment method and they are kept still up-to-date.

The concern pertaining to metalliferous antitumor medicines depends on that it is coincidentally found that metal complexes stop the cell division. Cisplatin (cis-diamminedichloroplatinum (II), cis-$Pt(NH_3)_2Cl_2$), known as an active substance in these compounds, has found a use in a short time and taken into certified medicine list by American Food and Drug Administration (FDA) thanks to its antitumor features. Nowadays, cisplatin administrated for the purpose of treatment on one of the two cancer patients is known effective particularly on ovarian cancer and testicular cancer patients. Cisplatin constitutes serious side effects such as nausea, vomiting, nephropathies, deformations in nervous system beside its chemotherapeutic effects. In many cases, it is possible that cisplatin has an insufficient response on tumor, i.e. that tumor reduces or eliminates anticancer efficiency. That the tumor exhibits resistance is a case that can be observed in a portion of other cancer medicines and this may vary according to the tissue, on which tumor is developed. However, it is frequently observed in the administration of cisplatin that tumor exhibits resistance to the medicine in many tissues among known drugs depending on the dosage and period, and this restricts use areas of the medicine and its tumor types. Severe side effects and acquired or settled cisplatin resistance lead to synthesizing various compounds to be administered in cancer treatments.

Phthalocyanines are studied widely because of its use in various scientific fields such as photocopy devices, chemical sensors, electro and photocatalysis processes, electrochromic applications and photodynamic therapy and cancer treatments as a photoconductive material in recent years, in addition to conventional uses as dye and pigments.

Silicon phthalocyanines have become outstanding compounds in recent years because they avoid aggregation resulting from macrocyclic $\pi$-system, reduce interaction between molecules and increase solubility, when compared to

derivatives of peripherally or non-peripherally substituted metallo phthalocyanine due to their axial substitution. Synthesis of water-soluble phthalocyanines is of important particularly for biological applications. Since peripheral tetra-substituted water-soluble phthalocyanines exhibit aggregation in the water, they offer lower efficiencies in biological applications. However, silicon phthalocyanines do not exhibit aggregation in the water due to its axial substitution. This feature makes silicon phthalocyanines and naphthalocyanines important for biological applications.

In the state of art, the patent application CN102327231 discloses a method for preparing water-soluble phthalocyanine silicon dioxide nanoparticles and for preparing photodynamic drug intravenous injection thereof.

The patent application CN1861603 (A) describes use of axial-substituted silicon phthalocyanines in tumor treatments as photo-synthesizer in the photodynamic therapy.

Another patent application US9890181 B2 discloses use of silicon phthalocyanines in the treatment of breast, melanoma, lung and liver cancers.

WO2018086242A1 relates to a pH-sensitive ketal-linked cholesterol-silicon phthalocyanine complex, a preparation method therefor and medical application thereof. Due to the presence of cholesterol groups, the series of complexes are hardly taken by tumor cells and normal cells; however, in a tumor tissue cell external subacid environment, ketal linkage undergoes hydrolysis reaction, and hydrolysis derivatives of silicon phthalocyanine can be easily taken by cancer cells.

Another patent application US2013237503A1 discloses pharmaceutical compositions comprising phosphaplatins, stable isolated monomeric phosphato complexes of platinum (II) and (IV) which can be used in the treatment of cisplatin- and carboplatin-resistant cancers.

WO2017000379A1 relates to a silicon phthalocyanine complex, and a preparation method and pharmaceutical application thereof. Th as a photosensitizer, especially for cancer treatments.

Another patent application CN105669735 (A) relates to an axially substitutive silicon phthalocyanine complex and a doxorubicin conjugate thereof and a preparation and application method thereof. The coupling agent has both a photodynamic therapy effect and a chemotherapy effect and can be prepared into a novel anti-cancer drug.

Considering studies conducted with the scope of the state of art, it is of importance that the solubility in water should be provided to be directly injected to body in terms of using synthesized compounds as drug. However, since phthalocyanines exhibit aggregation in the water, they offer lower efficiencies in biological applications, preventing that they are used as drugs.

In the study of Ipek Omeroglu et al, axially di-(alpha,alpha-diphenyl-4-pyridylmethoxy) silicon(IV) phthalocyanine (3) and its quaternized derivative (3Q) were synthesized and tested as photosensitizers against tumor and bacterial cells. The photodynamic efficacy of synthesized photosensitizers (3 and 3Q) were evaluated with promising photocytotoxicity (17% cell survival for 3 and 28% for 3Q) against the cervical cancer cell line (HeLa). The photodynamic inactivation of pathogenic bacterial strains Streptococcus mutans, Staphylococcus aureus, and Pseudomonas aeruginosa suggested a high susceptibility with quaternized derivative (3Q) [1].

Another patent no. CN 107474064A discloses positively charged water-soluble arm type branch ligand silicon phthalocyanine complex of one kind and its preparation method and application. The complex is applied to light inactivation multidrug resistant Bao Man not as photosensitizer Lever bacterium and treating cancer and cell imaging.

Considering explanations and studies above mentioned, it is synthesized silicon phthalocyanine compounds, whose effect spectrum is wider compared to metalliferous compounds and whose toxic effect is lower compared to metalliferous compounds and whose side effects are reduced and which can be directly injected to body by means of its water-soluble feature and which can be used as potential antitumor medicine and which have anticancer and antioxidant effects and which have functional groups of (3,5-bis{3-[3-(diethylamino)phenoxy]propoxy}phenyl)methoxy or (3,5-bis{3-[3-(dimethylamino)phenoxy]propoxy}phenyl)methoxy having in vitro cancer effects against cancer types of lung, liver, breast and melanoma.

## Objects of the Invention

It is an object of the invention to synthesize silicon phthalocyanine compounds, which constitute an activity in cancer cells resistant particularly to cisplatin and whose effect spectrum is wider compared to cisplatin and which have toxic effect less than cisplatin and whose side effects are reduced and which can be used as potential antitumor drug and which comprise anticancer and antioxidant effects and which have functional groups of (3,5-bis{3-[3-(diethylamino)phenoxy]propoxy}phenyl)methoxy or DNA-targeted (3,5-bis{3-[3-(dimethylamino)phenoxy]propoxy}phenyl)methoxy that is water-soluble.

It is another object of the invention to synthesize compound that affects human breast (BT-20), melanoma (SK-Mel 128), liver (SNU-398) and lung (A549) cancer cells and exhibits a cytotoxic activity that is effective on said lines.

It is another object of the invention to synthesize compound that exhibits DNA binding, DNA cleavage and topoisomerase I inhibitor effect and that can be used as antitumor drug.

It is another object of the invention to decrease foreign-source dependency by means of conducting pharmacokinetiand preclinical studies of active medicine candidate compounds and to implement promising novel treatment methods by employing our own resources.

It is another object of the present invention is to improve preclinical efficacies and pharmaco-safety of compounds having the potential of being a medication and their pharmacokinetic-pharmacodynamic and toxicological studies thereof in both in vitro and in vivo suitable models.

## Description of the Figures

**Figure 1** Absorption spectrum of (A) compound 7a and (B) compound 3a in the absence and presence of increasing amounts of CT-DNA.

**Figure 2** Competitive EB binding studies of (A) compound 7a and (B) compound 3a.

**Figure 3** Supercoiled pBR322 plasmid DNA hydrolytic nuclease activity and nuclease activity under irradiation of compound 7a.

**Figure 4** Oxidative nuclease activity of (A) compound 7a and (B) compound 3a without/with irradiation.

**Figure 5** Topoisomerase I inhibition effect of (A) compound 7a and (B) compound 3a.

**Figure 6** Topoisomerase II inhibition effect of compound 7a.

**Figure 7** Supercoiled pBR322 plasmid DNA hydrolytic nuclease activity of compound 3a.

**Figure 8** Supercoiled pBR322 plasmid DNA photonuclease activity of compound 3a.

**Figure 9** Determination of reactive oxygen types playing role in photonuclease activity of supercoiled pbr322 plasmid dna of compound 3a.

## Description of the invention

The invention relates to water-soluble silicon phthalocyanine compounds which comprises functional groups of (3,5-bis{3-[3-(diethylamino)phenoxy]propoxy}phenyl)methoxy or (3,5-bis{3-[3-(dimethylamino)phenoxy]propoxy}phenyl)methoxy and synthesizing method thereof.

Silicon phthalocyanine compound comprising groups of (3,5-bis{3-[3-(diethylamino)phenoxy]propoxy}phenyl)methoxy with a molecule name bis(3,5-bis{3-[3-(diethylmethylamino)phenoxy]propoxy}phenyl)methoxy)phthalocyaninato silicon (IV) Iodide (7a) which is synthesized is illustrated with Formula I.

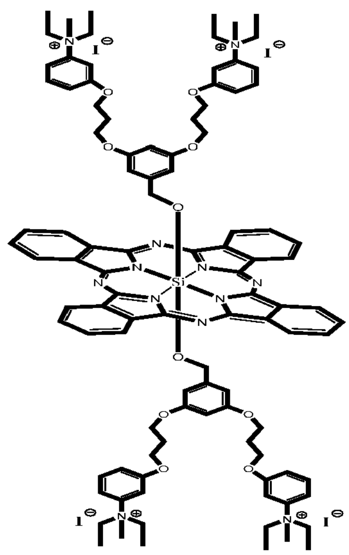

Formula I

Silicon phthalocyanine compound comprising groups of (3,5-bis{3-[3-(dimethylamino)phenoxy]propoxy}phenyl)methoxy with a molecule name bis(3,5-bis{3-[3-trimethylamino)phenoxy]propoxy}phenyl)methoxy)phthalocyaninato silicon (IV) Iodide (3a) which is synthesized is illustrated with Formula II.

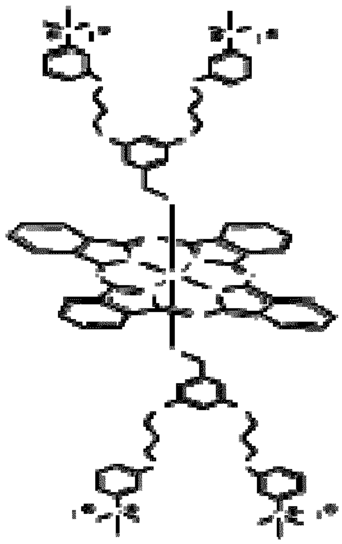

Formula II

The synthesized silicon phthalocyanine compounds related to the invention exhibits DNA binding, DNA cleavage and topoisomerase I inhibition effects. UV-Vis Absorption Titrations, EB Binding Studies competitive with UV, Competitive ethidium bromide binding study by UV-vis spectroscopy, Determination of DNA Melting Temperature, DNA Nuclease Activity, Hydrolytic Nuclease Activity, DNA Oxidative Cleavage Ability, Determination of Reactive Oxygen Types Playing Role in Oxidative Nuclease Activity, Topoisomerase I Inhibition, Cytotoxicity Studies are carried out so as to prove DNA Binding and DNA cleavage and topoisomerase I inhibition effects and the associated results thereof are shared.

## DNA Binding Experiments

### • UV-Vis Absorption Titration

UV-Vis absorption titration method is one of the most effective means in determining the mode of DNA-compound interactions.

Stock solutions of compounds are obtained by means of being solved in water and stored in a room temperature in darkness. Stock solutions of CT-DNA are prepared in 5 mM Tris-HCl buffer of 7.2 pH with 50 mM NaCl and stored at 4 °C for three days. At the end of this period, absorbance rates of 260 and 280 nm are calculated so as to determine rate of DNA impurity. That the rate is between 1.8 and 1.9 shows DNA impurity. Concentration of DNA is measured and calculated at 260 nm by using molar excitation coefficient of (ε) 6600 $M^{-1}cm^{-1}$. All studies with the interaction between compounds and CT-DNA are carried out in 5 mM Tris-HCl/50 mM NaCl (pH 7.2) by keeping stock solutions prepared in water at the stable concentration and changing CT-DNA concentration. While carrying out this process, absorbance of CT-DNA is eliminated by adding CT-DNA in an equal amount on the blind solution.

Variations in the absorbance intensity are presented in percent.

$$\%H = [(A_1 - A_2) / (A_1)] \times 100$$

$A_1$: Maximum absorbance of the compound; $A_2$: Absorbance of the compound subsequent to addition of DNA.

### • Competitive EB Binding Experiment

It is observed in competitive EB binding studies of compounds how the wave length and absorbance, in which EB (50 μM) whose concentration is kept stable presents the maximum absorbance, vary with addition of CT-DNA (50 μM) and then with simultaneous addition of DNA and complex. This study is carried out in the buffer of 5 mM Tris-HCl/50 mM NaCl (pH 7.2).

• **Determination of DNA Melting Temperature**

Studies pertaining to determination of DNA melting temperature is carried out by means of Cary 100 Bio UV-Visible Spectrophotometer with Varian Cary Temperature Controller unit.

The absorbance is measured at 260 nm at every 0.5 °C in the range of 60-90 °C by increasing temperature of the cell comprising the basin by 1 °C every minute by means of employing thermal melting program. Values $T_m$ are automatically calculated by the device from t(°C)-Absorbance curve of CT-DNA drawn in the presence and absence of the compound depending on its absorbance in 260 nm.

• **DNA Nuclease Activity**

Cleavage activities of supercoiled pBR322 plasmid DNA of the compounds are observed by means of using agarose-gel electrophoresis. When the supercoiled is exposed to pBR322 plasmid DNA electrophoresis, it is observed that the supercoiled form (Form I) migrates in the swiftest manner. In case of a cut occurred on a single chain, the supercoiled form creates a nicked form (Form II) moving in the slowest manner. In case both chains are cut, the linear form (Form III) migrating between Form I and Form II.

• **Hydrolytic Nuclease Activity of of pBR322 Plasmid DNA**

Total volume of reaction mixture contained 50 mM Tris-HCl (pH 7.0), supercoiled pBR322 plasmid DNA (250 ng), and different concentrations of compound. While the well content is loaded to 0.8 percent of agarose gel (comprising 0.25 μg/mL EB), gel loading dye also added and loaded to the gel. After addition of running buffer, it is exposed to electrophoresis by applying a current of 100 volt for 90 minutes. At the end of electrophoresis process, the gel is displayed under UV light and its image is saved.

• **Nuclease Activity of of pBR322 Plasmid DNA under irradiation**

It is used General Electric quartz lamb of 300 watts and optical filter of 700 nm for compounds illustrated as Formula I and II to light in the proper wave length so as to observe that the supercoiled pBR322 plasmid DNA is cleavage by the compounds and so as to evaluate usability potentials of the compounds in the photo-dynamic therapy. In this study, total volume of reaction mixture contained 50 mM Tris-HCl (pH 7.0), super-coiled pBR322 plasmid DNA (250 ng), and increasing concentrations of compounds. Afterwards, well contents are exposed to light for 15 minutes. At the end of this period, after mixtures are incubated in darkness at 37 °C for an hour, reactions are stopped by means of above-mentioned methods and cut products are observed on the agarose gel under UV light.

• **Oxidative Nuclease Activity of pBR322 Plasmid DNA**

Auxiliary reagents are added to $H_2O_2$, AA, ME medium so as to examine oxidative cleavage activities of the compounds.

In this study, pBR322 plasmid DNA is formed of buffer solution (50 mM Tris-HCl (pH 7)), auxiliary reagents and increasing concentrations of compounds, such that well contents are of 10 μL. The oxidative nuclease activity studies were performed under light irradiation. At the end of this period, after mixtures are incubated in darkness at 37 °C for an hour, reaction is stopped by means of above-mentioned methods and cleavage products are observed on the agarose gel under UV light.

• **Determination of Reactive Oxygen Types Playing Role in Oxidative Nuclease Activity of pBR322 Plasmid DNA**

Inhibition tests are performed in the presence of standard radical scavengers so as to determine active oxygen types effective on its cleavage in the presence of light on pBR322 plasmid DNA of complexes. For that purpose, it used ethanol (EtOH), methanol (MeOH) and dimethyl sulphoxide (DMSO) as hydroxyl radical scavenger; sodium azide and L-histidine as singlet oxygen scavenger; SOD as superoxide radical scavenger, EDTA as chelating agent and catalase as hydrogen peroxide adsorbent. It is also observed whether or not results obtained by using different types scavenging the same type of radicals support each other. Different reaction mixtures are formed by means of adding pBR322 plasmid DNA and complex in the efficient concentration and any one of above mentioned standard radical scavengers in variable concentrations into 50 mM Tris-HCl buffer. Subsequent to incubation of different reaction mixture formed at 37°C for an hour, it comprises the process of examining and monitoring these products.

• **Topoisomerase I Inhibition**

The buffer solution [35 mM Tris-HCl (pH 8.0), 72 mM KCl, 5 mM $MgCl_2$, 5 mM DTT, 5 mM spermidine, %0.1 Albumin] is formed of topoisomerase I enzyme of a unit and compounds of increasing concentrations under proper conditions of pBR322 plasmid DNA for topoisomerase I inhibition of the compounds. After incubation process, samples are loaded to 0.8 % of agarose gel, exposed to electrophoresis in the running buffer by applying 40 volt of current for 3 hours and forms obtained are observed on the agarose gel under UV light.

• **Topoisomerase II Inhibition**

The buffer solution [35 mM Tris-HCl (pH 8.0), 72 mM KCl, 1 mM ATP, 5 mM $MgCl_2$, 5 mM DTT, 5 mM spermidine, %0.1 Albumin] is formed of topoisomerase II enzyme of a unit and increasing concentrations of compounds under proper conditions of pBR322 plasmid DNA for topoisomerase II inhibition of the compounds. After incubation process, samples are loaded to 0.8 % of agarose gel, exposed to electrophoresis in the running buffer by applying 40 volts of current for 3 hours and forms obtained are observed on the agarose gel under UV light.

• **Cytotoxicity Tests (Experimental Part)**

Cytotoxic effects of test substances are testes by means of using human breast (BT-20), melanoma (SK-Mel 128), prostate (DU-145), liver (SNU-398) and lung (A549) cancer cell lines. Toxic effects of test substances on the healthy normal cells are studied on human fibroblast cells (HFC).

Cell suspension prepared from alpha-MEM having 10% FBS (Fetal Bovine Serum), fungizone and penicillin/streptomycin is distributed as $5\times10^3$ of cell in each well in the cell culture containers comprising 96 wells for the test. After being kept in a humid environment at 5% of $CO_2$ at 37 °C for 24 hours, solutions prepared as 600 $\mu$g/mL in the alpfa-MEM medium comprising 10% FBS penicillin/streptomycin and fungizone of Cisplatin as test substances and anti-neoplastic control drug are distributed in wells by performing 3-fold swift dilution. Cells are kept in a humid environment at 5% of $CO_2$ at 37°C for 48 hours in the presence of the test substance, of which final densities are of 200-0.09 $\mu$g/mL. 10 $\mu$L from the MTT solution (3-[4,5-dimethyltioazol-2,5-diphenyltetrazolium bromide) prepared at a density of 5 mg/mL is added to each well so as to determine the cell generation rate at the end of incubation period. After culture containers are kept in the humid environment at 5% of $CO_2$ at 37°C for 2 hours, the content is emptied and dimethyl sulphoxide (DMSO) is added in the value of 100 $\mu$L/well on cells so as to solubilize intracellular formazan crystals. After incubation for 30 minutes, the color intensity is measured in the wave length of 570/620 by means of ELISA Microplate Reader (Tecan, Sunrise).

Toxic effect percentages of test substances on cells at each density will be calculated separately according to the vitality (Test Optical Density/Control Optical Density x100) formula; test substance concentration forming 50% cytotoxicity (cell cytotoxicity50, $CC_{50}$) is calculated with the non-linear dose-response curve by means of GraphPad software program.

SI (Normal cell toxic effect/Cancer cell toxic effect) is determined by comparing the selective toxic effect (selective toxicity index =SI) of test substances on cancer cells to the toxic effect of the test substance on normal human cell (human fibroblast cell = HFC).

Cytotoxic effect of the compound of the synthesized water-soluble silicium-phthalocyanine is examined in vitro on human breast (BT-20), melanoma (SK-Mel 128), Liver (SNU-398) and lung (A549) cancer cell lines. As a result of studies, it is found to be more effective compared to cis-platin used standardly in BT-20, SK-Mel 128, SNU-398 and A549 cell lines and thereby, it is proved in in-vitro studies to have an usability potential in the development of anti-cancer drug.

**DNA Binding Studies (RESULTS)**

• **UV-Vis Absorption Titration**

Absorption spectrum of the silicium-phthalocyanine compounds examined in the presence and absence of CT-DNA informs us on the binding mode of compounds to DNA.

Binding of compounds to CT-DNA examined in the study is determined by measuring UV/Vis absorptions. Figure 1 shows absorption spectra in absence of DNA of compounds related to the invention and in existence of residual DNA concentration. As can be seen in Figure 1, the concentration-increased CT-DNA solution is added on the solution of compounds kept at the stable concentration and changes in the absorption spectra is saved. It is observed in the presence of CT-DNA that the compound 7a exhibits 43.64% of hypochromism and 6 nm of red-shift; compound 3a exhibits 25.40% of hypochromism and 6 nm of red-shift. This shows that compounds interact with CT-DNA in the type

of intercalation.

• **Competitive EB Binding Experiment**

In the light of information obtained as a result of UV-Vis absorption titration, it is supported by means of competitive binding studies conducted by using EB known to be an intercalative agent that the compound 7a interacts with DNA through intercalation. In a manner that it is going to be in line with the information obtained from the literature as a result of the test carried out, it is observed that not only EB's absorbance decreases due to the interaction with CT-DNA but also exhibits more redshift. Figure 2 shows competitive EB binding studies of compound 7a and 3a. As shown in Figure 2, as a result of addition of compound 7a and 3a, different rates of increase at the absorption density is observed such that these substances compete with EB so as to bind with CT-DNA.

• **Determination of DNA Melting Temperature**

The absorbance is measured at 260 nm at every 0.5 °C in the range of 60-90 °C by increasing temperature of the cell comprising the basin by 1 °C every minute by means of employing thermal melting program of DNA melting temperature of the compound 3a. Values Tm are automatically calculated by the device from t(°C)-Absorbance curve of CT-DNA drawn in the presence and absence of 3a depending on its absorbance in 260 nm. While the melting temperature of CT-DNA is at 75.57 °C, the melting temperature of the compounds CT-DNA + 3a is at 81.82 °C. An increase at the melting temperatures strengthens the potential that the compounds becomes an intercalative agents.

• **DNA Nuclease Activity**

DNA cleavage activities of the compounds 7a and 3a used in the study are revealed by means of agarose gel electrophoresis. Nuclease activities of the compounds are detected by means of conversion of DNA's Form I (supercoiled form) into Form **II** (nicked form) or Form III (linear form).

• **Hydrolytic Nuclease Activity and Nuclease Activity of pBR322 Plasmid DNA under irradiation**

Hydrolytic cleavage test is carried out by way of hydrolyzing DNA phosphodiester bonds so as to evaluate the nuclease activity of compounds **7a and** 3a. Figure 3 shows views of hydrolytic nuclease activities of the compounds 7a. The hydrolytic nuclease activity is observed for the compound **7a** depending on the concentration. It is expected that the compound **7a** exhibits an effective nuclease activity under irradiation because of its photosensitizer features. The compound **7a** exhibits the nuclease activity depending on the concentration. Figure 3 shows Band 1, DNA control; band2, DNA + **7a** (6.25 μM); band 3, DNA + **7a** (12.5 μM); band 4, DNA + **7a** (25 μM); band 5, DNA + **7a** (50 μM); band 6, DNA control; band 7, DNA + **7a** (6.25 μM); band 8, DNA + **7a** (12.5 μM); band 9, DNA + **7a** (25 μM); band 10, DNA + **7a** (50 μM). Figure 7 shows views of hydrolytic nuclease activities of the compound 3a. The hydrolytic nuclease activity is observed for the compound 3a depending on the concentration. Figure 7 shows Band 1, DNA control; band 2, DNA + 3a (12.5 μM); band 3, DNA + 3a (25 μM); band 4, DNA + 3a (50 μM); band 5, DNA + 3a (100 μM). It is examined whether stimulation of the mixture formed in the nuclease activity by the compound 3a of the superhelix pBR322 plasmid DNA with the light constitutes any contribution or not. It is expected as a result of the analysis that the compound 3a exhibits an effective nuclease activity in the presence of light because of its photosensitizer features. The compound 3a exhibits the nuclease activity depending on the concentration. It is observed that the compound 3a at the concentration of 100 μM destroys pBR322 plasmid DNA completely. Figure 8 shows views of superhelix pBR322 plasmid DNA photonuclease activity. Figure 8 shows Band 1, DNA control; band 2, DNA + 3a (12.5 μM); band 3, DNA + 3a (25 μM); band 4, DNA + 3a (50 μM); band 5, DNA + 3a (100 μM).

• **Oxidative Nuclease Activity of pBR322 Plazmid DNA**

Subsequent to the determination of hydrolytic nuclease activities of the compounds 7a and 3a, cleavage reactions are carried out by way of adding hydrogen peroxide ($H_2O_2$) (0.4 M), ascorbic acid (AA) (2.5 mM) and mercaptoethanol (ME) (0.4 M) so as to examine its oxidative cleavage capabilities with/without irradiation and the related results are given in Figure 4.

In accordance with the results, it is observed that effect of the compound **7a** is increased with the addition of oxidative agent under irradiation. Under irradiation, it is observed that it exhibits a significantly high cleavage activity. Figure 4-A shows Band 1: DNA control; band 2: DNA + **7a** (6.25 μM) + $H_2O_2$ (0.4 M); band 3: DNA + **7a** (12.5 μM) + $H_2O_2$ (0.4 M); band 4: DNA + **7a** (25 μM) + $H_2O_2$ (0.4 M); band 5: DNA + **7a** (6.25 μM) + $H_2O_2$ (0.25 M); band 6: DNA + **7a** (12.5 μM) + AA (0.25 mM); band 7: DNA + **7a** (25 μM) + AA (0.25 mM); band 8: DNA + **7a** (6.25 μM) + ME (0.4

M); band 9: DNA + **7a** (12.5 μM) + ME (0.4 M); band 10: DNA + **7a** (25 μM) + ME (0.4 M). Figure 4-B shows Band 1: DNA control; band 2: DNA + 3a (12.5 μM) + H2O2 (0.4 M); band 3: DNA + 3a (25 μM) + H2O2 (0.4 M); band 4: DNA + 3a (50 μM) + H2O2 (0.4 M); band 5: DNA + 3a (12.5 μM) + AA (0.25 mM); band 6: DNA + 3a (25 μM) + AA (0.25 mM); band 7: DNA + 3a (50 μM) + AA (0.25 mM); band 8: DNA + 3a (12.5 μM) + ME (0.4 M); band 9: DNA + 3a (25 μM) + ME (0.4 M); band 10: DNA + 3a (50 μM) + ME (0.4 M).

• **Determination of Reactive Oxygen Types Playing Role in Photonuclease Activity of pBR322 Plasmid DNA**

By means of stimulation of the compound 3a with the light, so as to research the possible mechanism of the DNA nuclease activity, reactions are carried out in the presence of hydroxyl radical scavengers (EtOH, MeOH and DMSO), singlet oxygen cleaners (NaN3 and L-Histidine), superoxide radical scavengers (SOD), hydrogen peroxide adsorbent (catalase) and chelating agent (EDTA) under the conditions of detection of hydrolytic cleavage activity. Figure 6 shows the gel image obtained. It is determined that other agents increase the cleavage, while the agents NaN3 and L-Histidine serving as the singlet oxygen scavenger for the compound 3a prevent the cleavage. Figure 6 shows Band 1: DNA control; band 2: DNA + 3a (50 μM) + ethanol; band 3: DNA + 3a (50 μM) + methanol; band 4: DNA + 3a (50 μM) + DMSO; band 5: DNA + 3a (50 μM) + NaN3; band 6: DNA + 3a (50 μM) + L-Histidine; band 7: DNA + 3a (50 μM) + SOD; band 8: DNA + 3a (50 μM) + Katalase ; band 8: DNA + 3a (50 μM) + EDTA.

• **Topoisomerase I Inhibition**

Effect of the compounds 7a and 3a against topoisomerase I enzyme is examined in accordance with relaxation method on pBR322 plasmid DNA and the related results are given in Figure 5. Nicked form is reduced in the presence of compounds examined and the supercoiled form rate increases. As can be seen in the results, the compound concentration increasing for the compound 7a as well as image density of the supercoiled pBR322 plasmid DNA increase. Figure 5-A shows Band 1: DNA control; band 2: DNA + 1 U Topo I band 3: DNA + 1 U Topo I + **7a** (12.5 μM); band 4: DNA + 1 U Topo I + **7a** (25 μM); band 5: DNA + 1 U Topo I + **7a** (37.5 μM); band 6: DNA + 1 U Topo I + irinotecan (12.5 μM); band 7: DNA + 1 U Topo I + irinotecan (25 μM); band 8: DNA + 1 U Topo I + irinotecan (37.5 μM) + band 9: DNA + 1 U Topo I + irinotecan (50 μM). Figure 5-B shows Band 1: DNA control; band 2: DNA + 1 Unit topoisomerase; band 3: DNA + 1 Unit topoisomerase + 3a (12.5 μM); band 4: DNA + 1 Unit topoisomerase + 3a (12.5 μM); band 5: DNA + 1 Unit topoisomerase + 3a (25 μM).

• **Topoisomerase II Inhibition**

Effect of the compound **7a** against topoisomerase II enzyme is examined in accordance with relaxation method on pBR322 plasmid DNA and the related results are given in Figure 6. Effective results are observed compared to doxorubicin used as a standard compound even in the presence of compounds examined. FIGURE 6 shows Band 1: DNA control (Tris-HCl pH 8); band 2: DNA (in buffer for topoisomerase II); band 3: DNA + 1 U Topo II; band4: DNA + 1 U Topo II **+7a** (12.5 μM); band 5: DNA + 1 U Topo I + **7a** (25 μM); band 6: DNA + 1 U Topo I + **7a** (37.5 μM); band 7: DNA + 1 U Topo II + doxorubicin (12.5 μM); band 8: DNA + 1 U Topo II + doxorubicin (25 μM); band 9: DNA + 1 U Topo II + doxorubicin (37.5 μM) + band 10: DNA + 1 U Topo II + doxorubicin (50 μM) .

• **Cytotoxicity Studies (Results)**

The cytotoxicity results of the compound of Bis(3,5-bis{3-[3-(diethlymethylamino)phenoxy]propoxy}phenyl)methoxy)phthalocyaninato silicon (IV) Iodide (7a) and Bis(3,5-bis{3-[3-trimethylamino)phenoxy]propoxy}phenyl) methoxy)phthalocyaninato silicon (IV) Iodide (3a) which are performed against human breast (BT-20), melanoma (SK-Mel 128), prostate (DU-145), liver (SNU-398) and lung (A549) cancer cell lines are given in Table 1. In this study, cis-platin is used as positive control. According to the results, it is detected that both compounds comprises in vitro activity greater than cis-platin commonly used in clinic on BT-20, SK-Mel 128, SNU-398, DU-145 and A549 cell lines.

**Table 1.** $CC_{50}$ (μM) values of the compound 7a and 3a against *in vitro* A549, BT20, SNU-398, DU-145 ve SK-MEL 128 cancer lines

| Code | A549 | BT20 | SNU-398 | DU-145 | SK-MEL 128 |
|---|---|---|---|---|---|
| **7a** | 1.18 | 1.44 | 0.49 | 2.99 | 1.13 |
| **3a** | 2.24 | 3.96 | 0.86 | 4.63 | 1.47 |
| **Cis-platin** | 9.66 | 21.99 | 4.33 | 1.66 | 13.99 |

It is possible to directly inject into the body comprising cells with anti-tumor by means of the water-soluble feature of the obtained compound.

The compound of bis(3,5-bis{3-[3-(diethlymethylamino)phenoxy]propoxy}phenyl)methoxy)phthalocyaninato silicon(IV) Iodide (7a) can be generally obtained in three (3) stages. These stages are:

**1. Stage:** Synthesizing (3,5-bis{3-[3-(diethylamino)phenoxy]propoxy}phenyl) methanol compound,

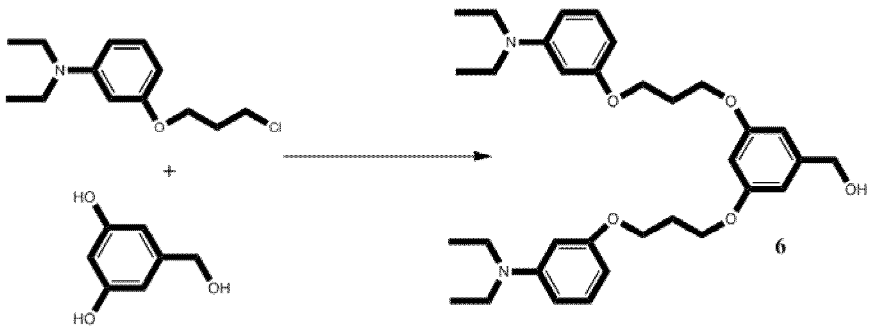

6

**2. Stage:** Synthesizing the compound of bis(3,5-bis{3-[3-(diethlyamino) phenoxy]propoxy}phenyl)methoxy)phthalocyaninato silicon(IV) by use of the compound of (3,5-bis{3-[3-(diethylamino)phenoxy]propoxy}phenyl)methanol synthesized in the first stage.

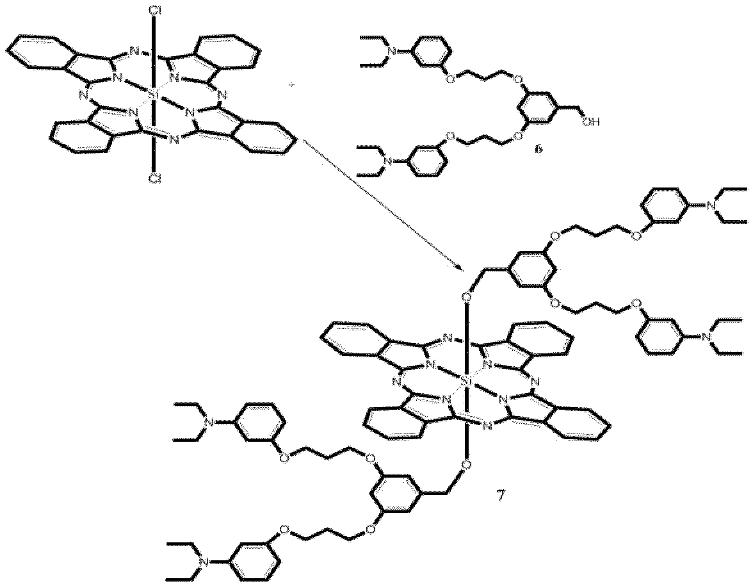

6

7

**3. Stage:** Synthesizing bis(3,5-bis{3-[3-(diethlymethylamino) phenoxy]propoxy}phenyl)methoxy)phthalocyaninato silicon(IV) Iodide (7a) by use of the compound of bis(3,5-bis{3-[3-(diethlyamino)phenoxy]propoxy}phenyl)methoxy)phthalocyaninato silicon(IV) synthesized in the second stage.

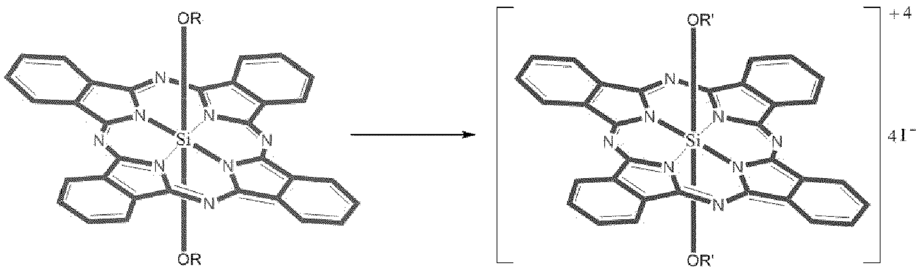

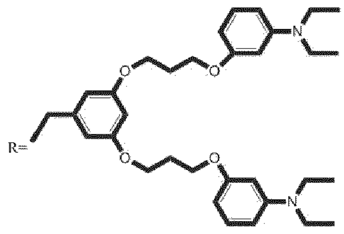

7

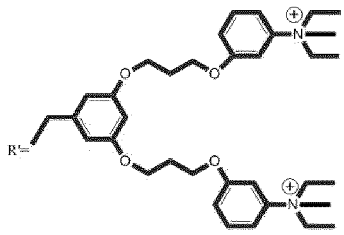

7a

1. Stage: Synthesizing (3,5-bis{3-[3-(diethylamino)phenoxylpropoxy}phenyl)methanol compound;

- Dissolving in 100-150 mL of acetone by adding 3,5-dihydroxybenzylalcohol and dry $K_2CO_3$ into single-necked flask,
- Removing the dissolved oxygen of the system for a couple of times in the nitrogen atmosphere by adding [3-(3-chloropropoxy)phenyl]diethylamine and 18-crown-6 on the mixture dissolved,
- Mixing the reaction mixture in the nitrogen atmosphere at 70 °C for 2 days,
- Evaporating completely the mixture cooled down to the room temperature,
- Dissolving the product remained at the end of evaporation in the 45-60 mL of chloroform and extracting for more than one in 25-40 mL of pure water,
- Evaporating the chloroform by drying the organic phase subsequent to the extraction over $MgSO_4$,
- Purifying the obtained crude product from the aluminum oxide loaded column with the solvent $CHCl_3$ and obtaining the compound of (3,5-bis{3-[3-(diethylamino)phenoxy]propoxy}phenyl)methanol being a fawn colored oily substance. The obtained (3,5-bis{3-[3-(diethylamino)phenoxy]propoxy}phenyl)methanol is depicted with Formula (III).

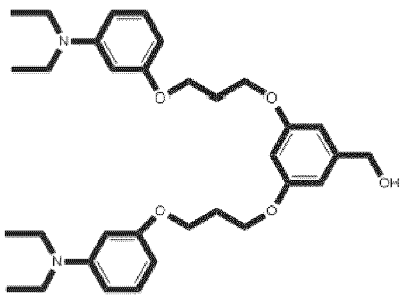

(III)

3,5-dihydroxybenzylalcohol used in synthesizing the compound of bis{3-[3-(diethylamino)phenoxy]propoxy} phenyl)methanol is of 1.21 g, 8,61 mmol. Dry $K_2CO_3$ is of 8.09 g, 58.8 mmol.

[3-(3-chloropropoxy)phenyl]diethylamine is of 3.96 g, 16.43 mmol and 18-crown-6 is of 1.76 g, 6.65 mmol.

2. Stage Synthesizing the compound of bis(3,5-bis{3-[3-diethylamino)phenoxy]propoxy}phenyl)methoxy)phthalocyanine silicon (IV)

- Dissolving in 10 mL of toluene by adding silicon phthalocyanine dichloride (100 mg, 0.16 mmol), (3,5-bis{3-[3-(diethylamino)phenoxy]propoxy}phenyl)methanol (171 mg, 0.32 mmol)
- Mixing at the room temperature in the nitrogen atmosphere for 10 minutes,
- Removing the dissolved oxygen of the system for a couple of times in the nitrogen atmosphere by swiftly adding NaH (7.85 mg, 0.32 mmol) into the obtained mixture,
- Mixing in the nitrogen atmosphere at 120 °C for 24 hours,
- Evaporating completely the solvent of the mixture cooled down to the room temperature under low pressure,

Purifying the obtained crude product with the solvent $CHCl_3$ by using the aluminum oxide filling material from the column and obtaining the compound of bis(3,5-bis{3-[3-diethylamino)phenoxy]propoxy}phenyl)methoxy) phthalocyaninato silicon(IV). The obtained compound of bis(3,5-bis{3-[3-diethylamino)phenoxy]propoxy}phenyl)methoxy)phthalocyaninato silicon(IV) is depicted with Formula (IV).

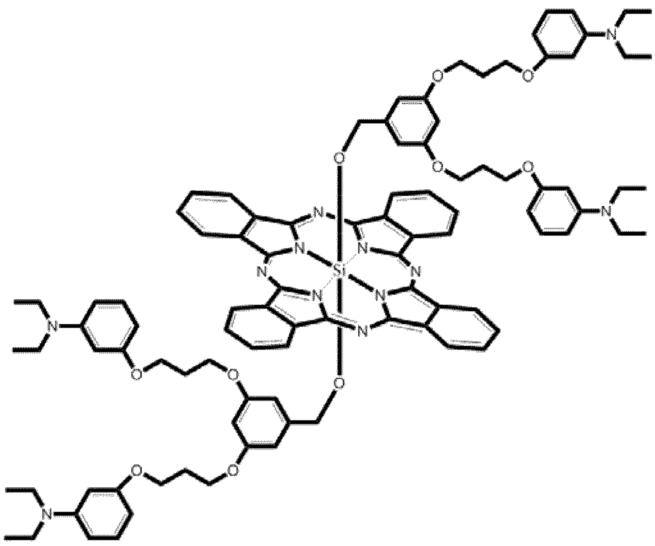

IV

3. Stage: Synthesizing the compound of bis(3,5-bis{3-[3-(diethlyamino)phenoxy]propoxy}phenyl)methoxy)phthalocyaninato silicon(IV) Iodide (7a)

- Dissolving in 4 mL of chloroform by putting the compound of bis(3,5-bis{3-[3-(diethlyamino)phenoxy]propoxy}phenyl)methoxy)phthalocyaninato silicon(IV) into 50 mL of a single necked flask (40 mg, 0.024 mmol),
- Mixing at the room temperature for 4 days in a lid-closed manner by adding 5 mL of methyl iodide thereon,
- Washing firstly with chloroform, acetone and lastly with diethyl ether by filtering the product precipitated in the medium and drying in vacuum oven and obtaining the water-soluble compound **(7a)** of bis(3,5-bis{3-[3-diethylmethylamino)phenoxy]propoxy}phenyl)methoxy)phthalocyaninato silicon(IV) Iodide. The obtained water-soluble compound **(7a)** of bis(3,5-bis{3-[3-(diethlymethylamino)phenoxy]propoxy}phenyl)methoxy)phthalocyaninato silicon(IV) Iodide is depicted with Formula (I).

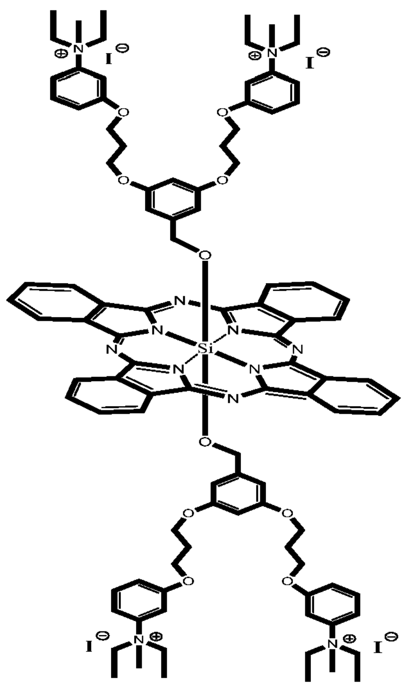

(I)

The compound of bis(3,5-bis{3-[3-trimethylamino)phenoxy]propoxy}phenyl)methoxy)phthalocyaninato silicon(IV) Iodide (3a) can be generally obtained in three (3) stages. These stages are:

**1. Stage:** Synthesizing (3,5-bis{3-[3-(dimethylamino)phenoxy]propoxy}phenyl) methanol compound,

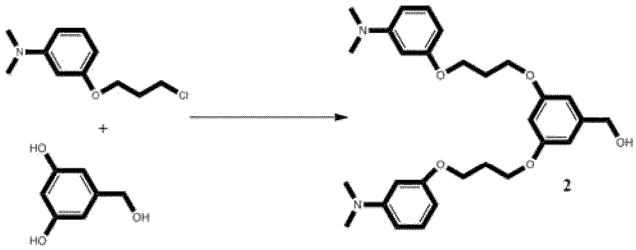

2

**2. Stage:** Synthesizing the compound of bis(3,5-bis{3-[3-(dimethlyamino) phenoxy]propoxy}phenyl)methoxy)phthalocyaninato silicon(IV) by use of the compound of (3,5-bis{3-[3-(dimethylamino)phenoxy]propoxy}phenyl)methanol synthesized in the first stage.

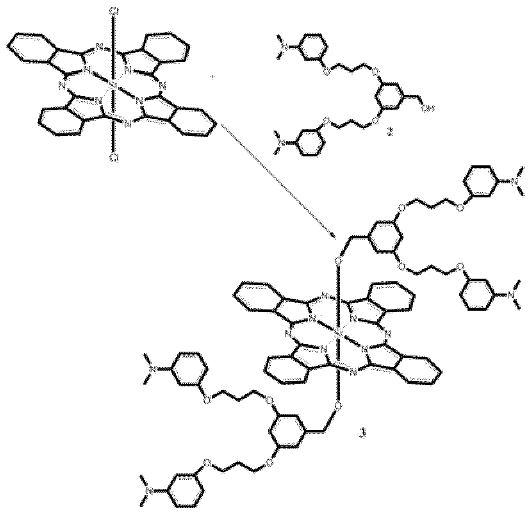

2

3

**3. Stage:** Synthesizing bis(3,5-bis{3-[3-trimethylamino) phenoxy]propoxy}phenyl)methoxy)phthalocyaninato silicon(IV) Iodide (3a) by use of the compound of bis(3,5-bis{3-[3-(dimethlyamino)phenoxy]propoxy}phenyl)methoxy)phthalocyaninato silicon(IV) synthesized in the second stage.

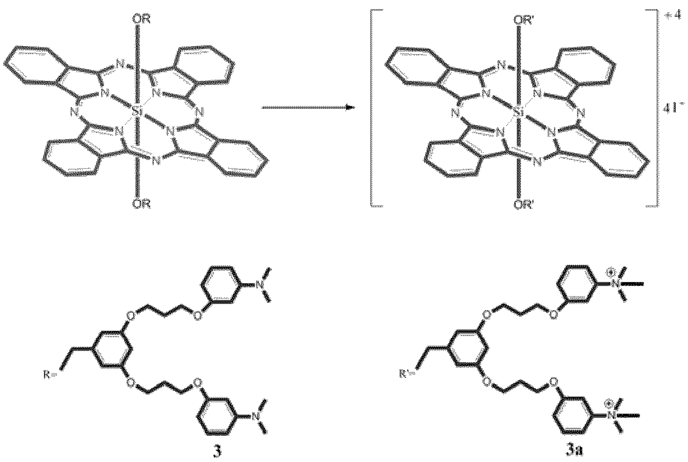

3 3a

1. Stage: Synthesizing (3,5-bis{3-[3-(diethylamino)phenoxy]propoxy}phenyl)methanol compound;

- Dissolving in 100-150 mL of acetone by adding 3,5-dihydroxybenzylalcohol and dry $K_2CO_3$ into single-necked flask,
- Removing the dissolved oxygen of the system for a couple of times in the nitrogen atmosphere by adding [3-(3-chloropropoxy)phenyl]dimethylamine and 18-crown-6 on the mixture dissolved,
- Mixing the reaction mixture in the nitrogen atmosphere at 70 °C for 2 days,
- Evaporating completely the mixture cooled down to the room temperature,
- Dissolving the product remained at the end of evaporation in the 45-60 mL of chloroform and extracting for more than one in 25-40 mL of pure water,
- Evaporating the chloroform by drying the organic phase subsequent to the extraction over $MgSO_4$,
- Purifying the obtained crude product from the aluminum oxide loaded column with the solvent $CHCl_3$ and obtaining the compound of (3,5-bis{3-[3-(dimethylamino)phenoxy]propoxy}phenyl)methanol being a fawn colored oily substance. The obtained (3,5-bis{3-[3-(dimethylamino)phenoxy]propoxy}phenyl)methanol is depicted with Formula (V).

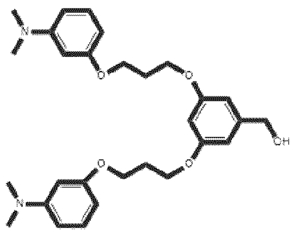

(V)

3,5-dihydroxybenzylalcohol used in synthesizing the compound of (3,5-bis{3-[3-(dimethylamino)phenoxy]propoxy}phenyl)methanol is of 1.18 g, 8,42 mmol. Dry $K_2CO_3$ is of 7.92 g, 57.43 mmol.
[3-(3-chloropropoxy)phenyl]dimethylamine is of 3.43 g, 16.08 mmol and 18-crown-6 is of 1.72 g, 6.51 mmol.

2. Stage Synthesizing the compound of bis(3,5-bis{3-[3-dimethylamino)phenoxy]propoxy}phenyl)methoxy)phthalocyanine silicon (IV)

- Dissolving in 10 mL of toluene by adding silicon phthalocyanine dichloride (125 mg, 0.2 mmol), (3,5-bis{3-[3-(dimethylamino)phenoxy]propoxy}phenyl)methanol (202 mg, 0.4 mmol)
- Mixing at the room temperature in the nitrogen atmosphere for 10 minutes,
- Removing the dissolved oxygen of the system for a couple of times in the nitrogen atmosphere by swiftly adding NaH (9,6 mg, 0.4 mmol) into the obtained mixture,
- mixing in the nitrogen atmosphere at 120 °C for 24 hours,
- Evaporating completely the solvent of the mixture cooled down to the room temperature under low pressure,

Purifying the obtained crude product with the solvent $CHCl_3$ by using the aluminum oxide filling material from the column and obtaining the compound of bis(3,5-bis{3-[3-dimethylamino)phenoxy]propoxy}phenyl)methoxy) phthalocyaninato silicon(IV). The obtained compound of bis(3,5-bis{3-[3-dimethylamino)phenoxy]propoxy}phenyl)methoxy)phthalocyaninato silicon(IV) is depicted with Formula (VI).

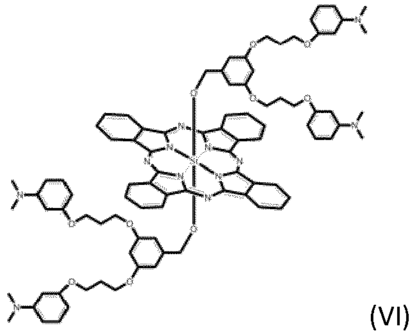

(VI)

3. Stage: Synthesizing the compound of bis(3,5-bis{3-[3-triethlyamino)phenoxy]propoxy}phenyl)methoxy)phthalocyaninato silicon(IV) Iodide (3a)

- Dissolving in 4 mL of chloroform by putting the compound of bis(3,5-bis{3-[3-dimethlyamino)phenoxy]propoxy}phenyl)methoxy)phthalocyaninato silicon(IV) into 50 mL of a single necked flask (40 mg, 0.026 mmol),
- Mixing at the room temperature for 4 days in a lid-closed manner by adding 5 mL of methyl iodide thereon,
- Washing firstly with chloroform, acetone and lastly with diethyl ether by filtering the product precipitated in the medium and drying in vacuum oven and obtaining the water-soluble compound of bis(3,5-bis{3-[3-trimethylamino)phenoxy]propoxy}phenyl)methoxy)phthalocyaninato silicon(IV) Iodide (3a). The obtained water-soluble compound of bis(3,5-bis{3-[3-trimethylamino)phenoxy]propoxy}phenyl)methoxy)phthalocyaninato silicon(IV) Iodide is depicted with Formula (II).

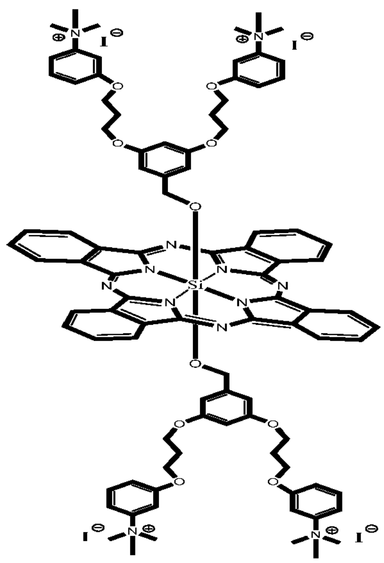

(II)

Subsequent to synthesizing the compound depicted with Formula (I) and (II), it is observed in studies conducted that it has a cytotoxicity activity in human breast and/or melanoma and/or liver and/or lung cancer cell lines. Since it is observed that anti-tumor drugs in the state of art have gastrointestinal problems and hematologic, neuronal, dermatological and cardiac side effects, a novel molecule that is demonstrated through the formula (I) (7a) and formula (II) (3a) which are oriented for alternative treatment strategies having safer adverse effect profile and target specific is proposed.

Method for synthesizing compounds of bis(3,5-bis{3-[3-(diethlymethylamino) phenoxy]propoxy}phenyl)methoxy)phthalocyaninato silicon(IV) Iodide (7a, Formula I) or bis(3,5-bis{3-[3-trimethylamino)phenoxy]propoxy}phenyl)methoxy) phthalocyaninato silicon(IV) Iodide (3a, Formula II) comprising below steps:

- Dissolving in 100-150 mL of acetone by adding 3,5-dihydroxybenzylalcohol and dry $K_2CO_3$ into single-necked flask,
- Removing the dissolved oxygen of the system for a couple of times in the nitrogen atmosphere by adding [3-(3-chloropropoxy)phenyl]diethylamine or [3-(3-chloropropoxy)phenyl]dimethylamine and 18-crown-6 on the mixture

dissolved,

- Mixing the reaction mixture in the nitrogen atmosphere at 70 °C for 2 days,
- Evaporating completely the mixture cooled down to the room temperature,
- Dissolving the product remained at the end of evaporation in the 45-60 mL of chloroform and extracting for more than one in 25-40 mL of pure water,
- Evaporating the chloroform by drying the organic phase subsequent to the extraction over $MgSO_4$,
- Purifying the obtained crude product from the aluminum oxide loaded column with the solvent $CHCl_3$ and obtaining the compound of (3,5-bis{3-[3-(diethylamino)phenoxy]propoxy}phenyl)methanol or (3,5-bis{3-[3-(dimethylamino)phenoxy]propoxy}phenyl)methanol being a fawn colored oily substance,
- Dissolving in 10 mL of toluene by adding silicon phthalocyanine dichloride (125 mg, 0.2 mmol), (3,5-bis{3-[3-(diethylamino)phenoxy]propoxy}phenyl)methanol (171 mg, 0.32 mmol) or silicon phthalocyanine dichloride (125 mg, 0.2 mmol), (3,5-bis{3-[3-(dimethylamino)phenoxy]propoxy} phenyl)methanol (202 mg, 0.4 mmol),
- Mixing at the room temperature in the nitrogen atmosphere for 10 minutes,
- Removing the dissolved oxygen of the system for a couple of times in the nitrogen atmosphere by swiftly adding NaH (9,6 mg, 0.4 mmol) into the obtained mixture,
- Mixing in the nitrogen atmosphere at 120 °C for 24 hours,
- Evaporating completely the solvent of the mixture cooled down to the room temperature under low pressure,
- Purifying the obtained crude product with the solvent $CHCl_3$ by using the aluminum oxide filling material from the column and obtaining the compound of bis(3,5-bis{3-[3-diethylamino)phenoxy]propoxy}phenyl)methoxy)phthalocyaninato silicon(IV) or bis(3,5-bis{3-[3-dimethylamino)phenoxy]propoxy} phenyl)methoxy)phthalocyaninato silicon(IV),
- Solving in 4 mL of chloroform by putting the compound of bis(3,5-bis{3-[3-diethylamino)phenoxy]propoxy}phenyl)methoxy) phthalocyaninato silicon(IV) or bis(3,5-bis{3-[3-dimethylamino)phenoxy]propoxy} phenyl)methoxy)phthalocyaninato silicon(IV) into 50 mL of a single necked flask (40 mg, 0.026 mmol),
- Mixing at the room temperature for 4 days in a lid-closed manner by adding 5 mL of methyl iodide thereon,
- Washing firstly with chloroform, acetone and lastly with diethyl ether by filtering the product precipitated in the medium and drying in vacuum oven and obtaining the water-soluble compound of bis(3,5-bis{3-[3-diethylmethylamino)phenoxy]propoxy}phenyl)methoxy) phthalocyaninato silicon(IV) Iodide (7a) or bis(3,5-bis{3-[3-trimethylamino)phenoxy]propoxy}phenyl)methoxy) phthalocyaninato silicon(IV) Iodide (3a).

**REFERENCES**

1. Ömeroğlu, I., Kaya, E. N., Göksel, M., Kussovski, V., Mantareva, V., & Durmus, M. (2017). Axially substituted silicon(IV) phthalocyanine and its quaternized derivative as photosensitizers towards tumor cells and bacterial pathogens. *Bioorganic* & *medicinal chemistry,* 25(20), 5415-5422. https://doi.org/10.1016/j.bmc.2017.07.065

## Claims

**1.** Water-soluble and non-aggregated silicon phtalocyanine compounds of Formula (I) or Formula (II) comprising functional groups of (3,5-bis{3-[3-(diethylamino)phenoxy]propoxy}phenyl)methoxy or (3,5-bis{3-[3-(dimethylamino)phenoxy]propoxy}phenyl)methoxy.

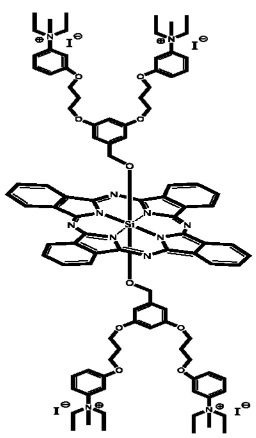

Formula (I)

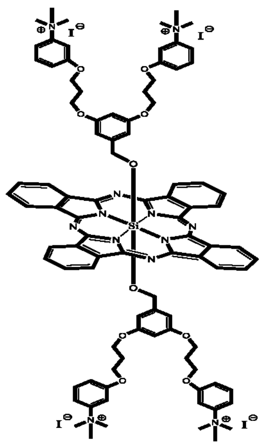

Formula (II)

**2.** A compound according to Claim 1 for use as a drug.

**3.** A compound according to Claim 1, having cytotoxicity activity on human breast (BT-20), melanoma (SK-Mel 128), liver (SNU-398) and lung (A549) cancer cell lines.

**4.** A method for synthesizing the compound of bis(3,5-bis{3-[3-(diethlymethylamino)phenoxy]propoxy}phenyl)methoxy) phthalocyaninato silicon(IV) Iodide (7a, Formula I) or bis(3,5-bis{3-[3-trimethylamino) phenoxy]propoxy}phenyl)methoxy)phthalocyaninato silicon(IV) Iodide (3a, Formula II), comprising below steps:

- Dissolving in 100-150 mL of acetone by adding 3,5-dihydroxybenzylalcohol and dry $K_2CO_3$ into single-necked flask,
- Removing the dissolved oxygen of the system for a couple of times in the nitrogen atmosphere by adding [3-(3-chloropropoxy)phenyl]diethylamine or [3-(3-chloropropoxy)phenyl]dimethylamine and 18-crown-6 on the mixture dissolved,
- Mixing the reaction mixture in the nitrogen atmosphere at 70 °C for 2 days,
- Evaporating completely the mixture cooled down to the room temperature,
- Dissolving the product remained at the end of evaporation in the 45-60 mL of chloroform and extracting for more than one in 25-40 mL of pure water,
- Evaporating the chloroform by drying the organic phase subsequent to the extraction over $MgSO_4$,
- Purifying the obtained crude product from the aluminum oxide loaded column with the solvent $CHCl_3$ and obtaining the compound of (3,5-bis{3-[3-(diethylamino)phenoxy]propoxy}phenyl)methanol or (3,5-bis{3-[3-(dimethylamino)phenoxy]propoxy}phenyl)methanol being a fawn colored oily substance,
- Dissolving in 10 mL of toluene by adding silicon phthalocyanine dichloride (125 mg, 0.2 mmol), (3,5-bis{3-[3-(diethylamino)phenoxy]propoxy}phenyl)methanol (171 mg, 0.32 mmol) or silicon phthalocyanine dichloride (125 mg, 0.2 mmol), (3,5-bis{3-[3-(dimethylamino)phenoxy]propoxy} phenyl)methanol (202 mg, 0.4 mmol),
- Mixing at the room temperature in the nitrogen atmosphere for 10 minutes,
- Removing the dissolved oxygen of the system for a couple of times in the nitrogen atmosphere by swiftly adding NaH (9,6 mg, 0.4 mmol) into the obtained mixture,
- Mixing in the nitrogen atmosphere at 120 °C for 24 hours,
- Evaporating completely the solvent of the mixture cooled down to the room temperature under low pressure,
- Purifying the obtained crude product with the solvent $CHCl_3$ by using the aluminum oxide filling material from the column and obtaining the compound of bis(3,5-bis{3-[3-diethylamino)phenoxy]propoxy}phenyl)methoxy)phthalocyaninato silicon(IV) or bis(3,5-bis{3-[3-dimethylamino)phenoxy]propoxy} phenyl)methoxy)phthalocyaninato silicon(IV),
- Dissolving in 4mL of chloroform by putting the compound of bis(3,5-bis{3-[3-diethylamino)phenoxy]propoxy} phenyl)methoxy) phthalocyaninato silicon(IV) or bis(3,5-bis{3-[3-dimethylamino)phenoxy]propoxy} phenyl)methoxy)phthalocyaninato silicon(IV) into 50 mL of a single necked flask (40 mg, 0.026 mmol),
- Mixing at the room temperature for 4 days in a lid-closed manner by adding 5 mL of methyl iodide thereon,
- Washing firstly with chloroform, acetone and lastly with diethyl ether by filtering the product precipitated in the medium and drying in vacuum oven and obtaining the water-soluble compound of bis(3,5-bis{3-[3-diethylmethylamino)phenoxy]propoxy}phenyl)methoxy) phthalocyaninato silicon(IV) Iodide (7a) or bis(3,5-bis{3-[3-trimethylamino)phenoxy]propoxy}phenyl)methoxy) phthalocyaninato silicon(IV) Iodide (3a).

**5.** A compound synthesized by the method according to Claim 4 for use in the treatment of human breast, melanoma, liver and/or lung cancer.

**6.** A compound according to Claim 1 for use in the treatment of human breast, melanoma, liver and/or lung cancer.

**Patentansprüche**

**1.** Wasserlösliche und nicht-aggregierte Silicium-Phtalocyaninverbindungen der Formel (I) oder der Formel (II), die funktionelle Gruppen von (3,5-Bis-{3-[3-(diethylamino)phenoxy]propoxy}phenyl)methoxy oder (3,5-Bis-{3-[3-(dimethylamino)phenoxy]propoxy}phenyl)methoxy umfassen.

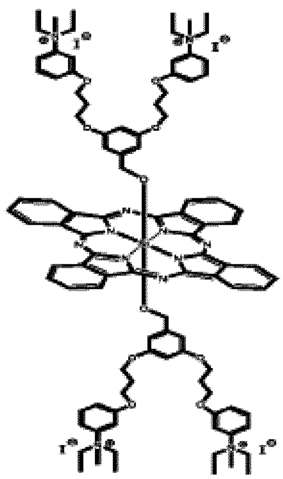

Formula (I)

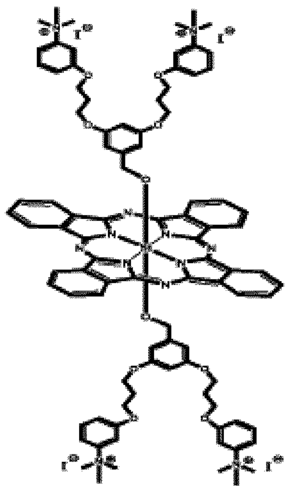

Formula (II)

**2.** Eine Verbindung nach Anspruch 1 zur Verwendung als Arzneimittel.

**3.** Verbindung nach Anspruch 1 mit zytotoxischer Aktivität auf menschliche Brust-(BT-20), Melanom- (SK-Mel 128), Leber- (SNU-398) und Lungen- (A549) Krebszelllinien.

**4.** Verfahren zur Synthese der Verbindung von Bis(3,5-bis{3-[3-(diethylmethylamino)phenoxy]propoxy}phenyl)methoxy)phthalocyaninato-Silicium(IV)-iodid (7a, Formel I) oder Bis(3,5-bis{3-[3-trimethylamino)phenoxy]propoxy}phenyl)methoxy)phthalocyaninato-Silicium(IV)-iodid (3a, Formel II), umfassend die folgenden Schritte:

- Auflösen in 100-150 mL Aceton durch Zugabe von 3,5-Dihydroxybenzylalkohol und trockenem $K_2CO_3$ in einem Einhalskolben,
- Entfernen des gelösten Sauerstoffs des Systems für ein paar Mal in der Stickstoffatmosphäre durch Zugabe von [3-(3-Chlorpropoxy)phenyl]diethylamin oder [3-(3-Chlorpropoxy)phenyl]dimethylamin und 18-Krone-6 auf die Mischung gelöst,
- Mischen der Reaktionsmischung unter Stickstoffatmosphäre bei 70 °C für 2 Tage,
- Durch vollständiges Verdampfen kühlte die Mischung auf Raumtemperatur ab,
- Auflösen des am Ende der Verdampfung verbliebenen Produkts in 45-60 mL Chloroform und Extraktion für mehr als eine in 25-40 mL reinem Wasser,
- Verdampfen des Chloroforms durch Trocknen der organischen Phase nach der Extraktion über $MgSO_4$,
- Reinigung des erhaltenen Rohprodukts aus der mit Aluminiumoxid beladenen Säule mit dem Lösungsmittel $CHCl_3$ und Gewinnung der Verbindung von (3,5-Bis{3-[3-(diethylamino)phenoxy]propoxy}phenyl)methanol oder (3,5-Bis{3-[3-(dimethylamino)phenoxy]propoxy}phenyl)methanol, die eine rehbraune ölige Substanz ist,
- Auflösen in 10 mL Toluol durch Zugabe von Siliciumphthalocyanindichlorid (125 mg, 0,2 mmol), (3,5-Bis{3-[3-(diethylamino)phenoxy]propoxy}phenyl)methanol (171 mg, 0. 32 mmol) oder Siliciumphthalocyanindichlorid (125 mg, 0,2 mmol), (3,5-Bis{3-[3-(dimethylamino)phenoxy]propoxy}phenyl)methanol (202 mg, 0,4 mmol),

• Mischen bei Raumtemperatur unter Stickstoffatmosphäre für 10 Minuten,

• Entfernen des gelösten Sauerstoffs aus dem System für einige Male in der Stickstoffatmosphäre durch schnelle Zugabe von NaH (9,6 mg, 0,4 mmol) in die erhaltene Mischung,

• Mischen unter Stickstoffatmosphäre bei 120 °C für 24 Stunden,

• Das Lösungsmittel der auf Raumtemperatur abgekühlten Mischung wird unter niedrigem Druck vollständig verdampft,

• Reinigen des erhaltenen Rohprodukts mit dem Lösungsmittel $CHCl_3$ unter Verwendung des Aluminiumoxid-Füllmaterials aus der Säule und Erhalten der Verbindung von Bis(3,5-bis{3-[3-diethylamino)phenoxy]propoxy} phenyl)methoxy)phthalocyaninato-Silicium(IV) oder Bis(3,5-bis{3-[3-dimethylamino)phenoxy]propoxy}phenyl)methoxy)phthalocyaninato-Silicium(IV), bis(3,5-bis{3-[3-diethylamino)phenoxy]propoxy}phenyl)methoxy)phthalocyaninato silicon(IV) or bis(3,5-bis{3-[3-dimethylamino)phenoxy]propoxy} phenyl)methoxy)phthalocyaninato silicon(IV),

• Lösen in 4 mL Chloroform durch Einbringen der Verbindung von Bis(3,5-bis{3-[3-diethylamino)phenoxy]propoxy}phenyl)methoxy)phthalocyaninato-Silicium(IV) oder Bis(3,5-bis{3-[3-dimethylamino)phenoxy]propoxy} phenyl)methoxy)phthalocyaninato-Silicium(IV) in 50 mL eines Einhalskolbens (40 mg, 0,026 mmol),

• Mischen bei Raumtemperatur für 4 Tage bei geschlossenem Deckel unter Zugabe von 5 mL Methyliodid,

• Waschen zunächst mit Chloroform, Aceton und zuletzt mit Diethylether, Filtrieren des im Medium ausgefallenen Produkts und Trocknen im Vakuumofen, wobei die wasserlösliche Verbindung Bis(3, 5-Bis{3-[3-diethylmethylamino)phenoxy]propoxy}phenyl)methoxy)phthalocyaninato -Silicium(IV)-iodid (7a) oder Bis(3,5-Bis{3-[3-trimethylamino)phenoxy]propoxy}phenyl)methoxy)phthalocyaninato-Silicium(IV)-iodid (3a).

**5.** Verbindung, die nach dem Verfahren gemäß Anspruch 4 synthetisiert wurde, zur Verwendung bei der Behandlung von menschlichem Brust-, Melanom-, Leber- und/oder Lungenkrebs.

**6.** Verbindung nach Anspruch 1 zur Verwendung bei der Behandlung von menschlichem Brust-, Melanom-, Leber- und/oder Lungenkrebs.

## Revendications

**1.** Composés de phtalocyanine de silicium solubles dans l'eau et non agrégés de formule (I) ou de formule (II) comprenant des groupes fonctionnels de (3,5-bis{3-[3-(diéthylamino)phénoxy]propoxy}phényl)méthoxy ou (3,5-bis{3-[3-(diméthylam ino)phénoxy]propoxy}phényl)méthoxy.

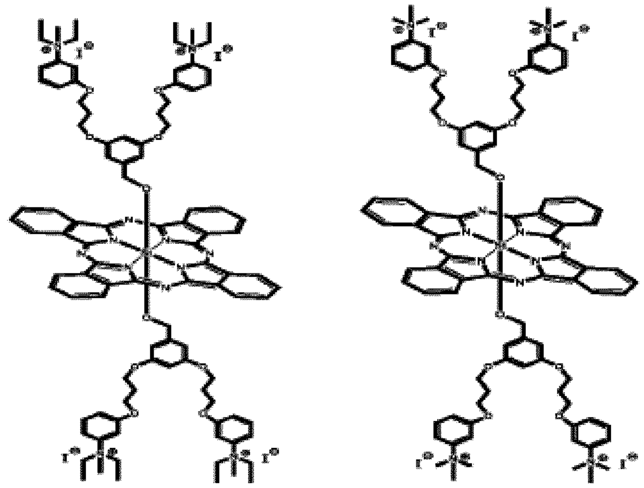

Formule (I) Formule (II)

**2.** Composé selon la revendication 1 destiné à être utilisé comme médicament.

3. Composé selon la revendication 1, ayant une activité cytotoxique sur les lignes cellulaires de cancer du sein (BT-20), du mélanome (SK-Mel 128), du foie (SNU-398) et du poumon (A549).

4. Procédé de synthèse du composé de bis(3,5-bis{3-[3-(diethlyméthylamino)phénoxy]propoxy}phényl)méthoxy) phtalocyaninato silicium (IV) Iodure (7a, formule I) ou bis(3,5-bis{3-[3-triméthylamino)phénoxy]propoxy}phényl)méthoxy)phtalocyaninatosilicium (IV) Iodure (3a, Formule II), comprenant les étapes ci-dessous :

• Dissoudre dans 100-150 mL d'acétone en ajoutant de l'alcool 3,5-dihydroxybenzylique et du $K_2CO_3$ sec dans un flacon à col unique,
• Éliminer l'oxygène dissous du système plusieurs fois dans l'atmosphère d'azote en ajoutant de la [3-(3-chloropropoxy)phényl]diéthylamine ou de la [3-(3-chloropropoxy)phényl]diméthylamine et du 18-couronne-6 sur le mélange dissous,
• Mélanger le mélange réactionnel sous atmosphère d'azote à 70°C pendant 2 jours,
• Evaporer complètement le mélange refroidi à température ambiante,
• Dissoudre le produit resté en fin d'évaporation dans 45 à 60 mL de chloroforme et extraire plus d'un dans 25 à 40 mL d'eau pure,
• Evaporer le chloroforme par séchage de la phase organique suite à l'extraction sur $MgSO_4$,
• Purifier le produit brut obtenu à partir de la colonne chargée d'oxyde d'aluminium avec le solvant $CHCl_3$ et obtenir le composé de (3,5-bis{3-[3-(diéthylamino)phénoxy]propoxy}phényl)méthanol ou (3,5-bis{3-[3-(diméthylamino)phénoxy]propoxy}phényl)méthanol étant une substance huileuse de couleur fauve,
• Dissoudre dans 10 mL de toluène en ajoutant du dichlorure de phtalocyanine de silicium (125 mg, 0,2 mmol), du (3,5-bis{3-[3-(diéthylamino)phénoxy]propoxy}phényl)méthanol (171 mg, 0,32 mmol) ou du silicium dichlorure de phtalocyanine (125 mg, 0,2 mmol), (3,5-bis{3-[3-(diméthylamino)phénoxy]propoxy}phényl)méthanol (202 mg, 0,4 mmol),
• Mélanger à température ambiante sous atmosphère d'azote pendant 10 minutes,
• Eliminer l'oxygène dissous du système plusieurs fois dans l'atmosphère d'azote en ajoutant rapidement NaH (9,6 mg, 0.4 mmol) au mélange obtenu,
• Mélanger sous atmosphère d'azote à 120 °C pendant 24 heures,
• Evaporer complètement le solvant du mélange refroidi à température ambiante sous basse pression,
• Purifier le produit brut obtenu avec le solvant $CHCl_3$ en utilisant le matériau de remplissage d'oxyde d'aluminium de la colonne et en obtenant le composé de bis(3,5-bis{3-[3-diéthylamino)phénoxy]propoxy} phényl)méthoxy)phtalocyaninato silicium(IV) ou de bis(3,5-bis{3-[3-diméthylamino)phénoxy]propoxy}phényl)méthoxy)phtalocyaninato silicium (IV),
• Dissoudre dans 4 mL de chloroforme en mettant le composé de bis(3,5-bis{3-[3-diéthylam ino)phénoxy]propoxy}phényl)méthoxy)phtalocyanine dans du silicium(IV) ou bis(3,5-bis{3-[ 3-diméthylamino)phénoxy]propoxy} phényl)méthoxy)phtalocyaninatosiliciu m(IV) dans 50 mL d'un flacon à col unique (40 mg, 0,026 mmol),
• Mélanger à température ambiante pendant 4 jours, couvercle fermé, en y ajoutant 5 mL d'iodure de méthyle,
• Laver d'une part au chloroforme, à l'acétone et enfin à l'éther diéthylique en filtrant le produit précipité dans le milieu et en séchant à l'étuve sous vide et en obtenant le composé hydrosoluble de bis(3,5-bis{3-[3-diéthylméthylamino)phénoxy]propoxy}phényl)méthoxy)phtalocyaninatos ilicium(IV) Iodure (7a) ou bis(3,5-bis{3-[3-triméthylamino)phénoxy]propoxy}phényl)méthoxy)phtalocyaninatosiliciu m (IV) Iodure (3a).

5. Composé synthétisé par le procédé selon la revendication 4 pour utilisation dans le traitement du cancer du sein, du mélanome, du foie et/ou du poumon chez l'humain.

6. Composé selon la revendication 1 destiné à être utilisé dans le traitement du cancer du sein, du mélanome, du foie et/ou du poumon chez l'humain.

A

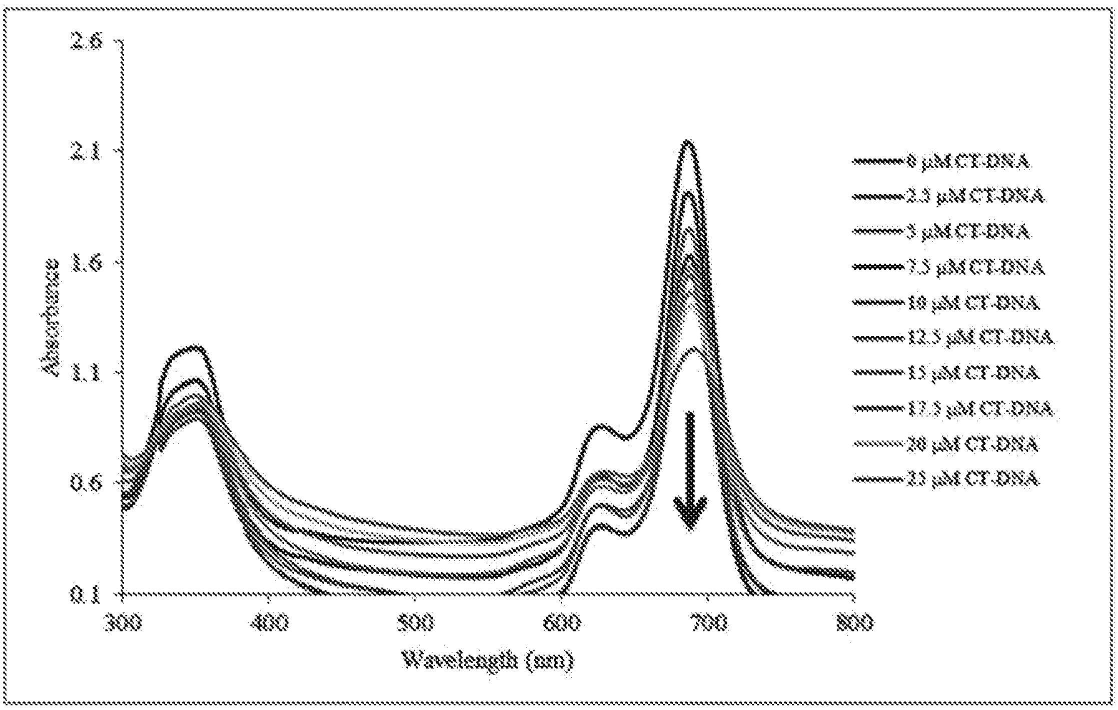
2.6
2.1
1.6
1.1
0.6
0.1
Absorbance
300
400
500
600
700
800
Wavelength (nm)
0 µM CT-DNA
2.5 µM CT-DNA
5 µM CT-DNA
7.5 µM CT-DNA
10 µM CT-DNA
12.5 µM CT-DNA
15 µM CT-DNA
17.5 µM CT-DNA
20 µM CT-DNA
25 µM CT-DNA

B

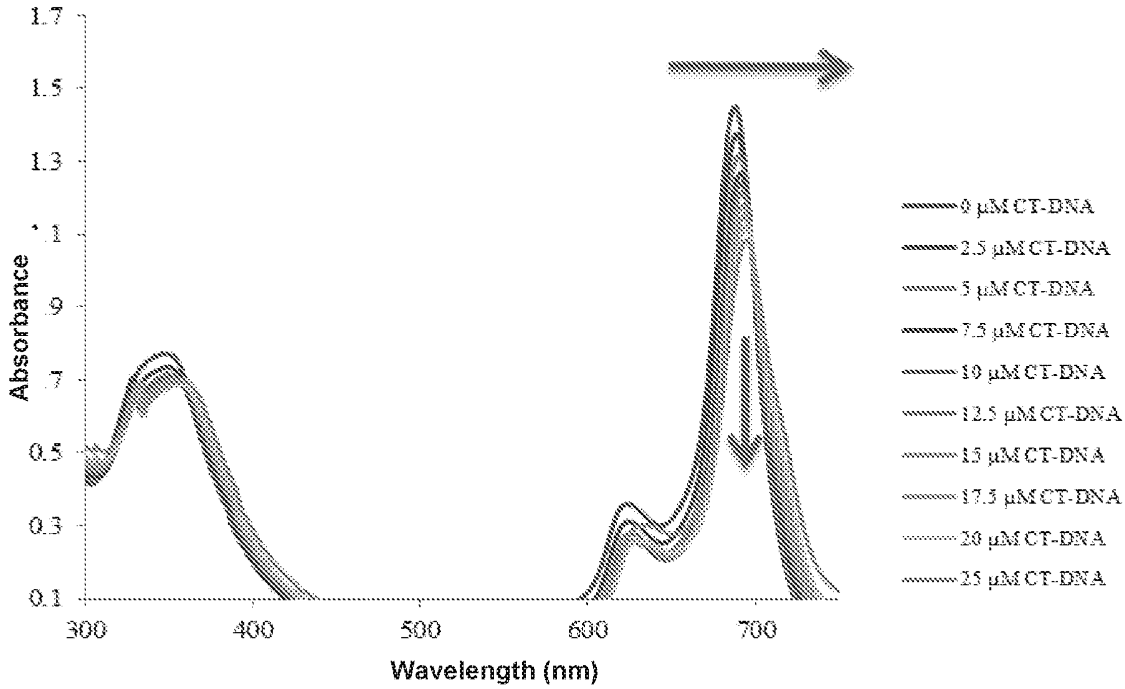
1.7
1.5
1.3
0.5
0.3
0.1
Absorbance
300
400
500
600
700
Wavelength (nm)
0 µM CT-DNA
2.5 µM CT-DNA
5 µM CT-DNA
7.5 µM CT-DNA
10 µM CT-DNA
12.5 µM CT-DNA
15 µM CT-DNA
17.5 µM CT-DNA
20 µM CT-DNA
25 µM CT-DNA

**Figure 1**

A

B

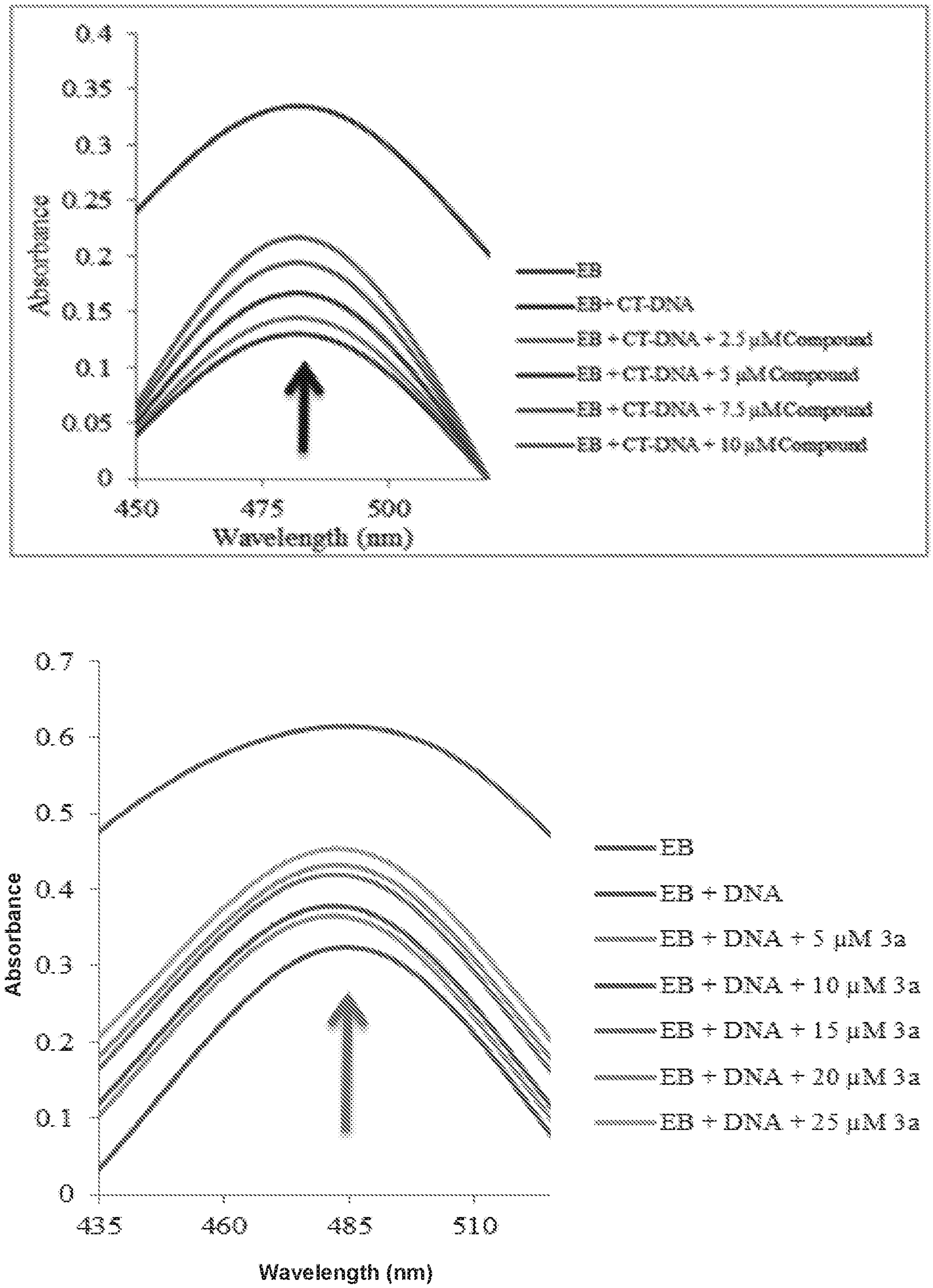
0.4
0.35
0.3
0.25
0.2
0.15
0.1
0.05
0
Absorbance
450
475
500
Wavelength (nm)
EB
EB+ CT-DNA
EB + CT-DNA + 2.5 μM Compound
EB + CT-DNA + 5 μM Compound
EB + CT-DNA + 7.5 μM Compound
EB + CT-DNA + 10 μM Compound
0.7
0.6
0.5
0.4
0.3
0.2
0.1
0
Absorbance
435
460
485
510
Wavelength (nm)
EB
EB + DNA
EB + DNA + 5 μM 3a
EB + DNA + 10 μM 3a
EB + DNA + 15 μM 3a
EB + DNA + 20 μM 3a
EB + DNA + 25 μM 3a

Figure 2

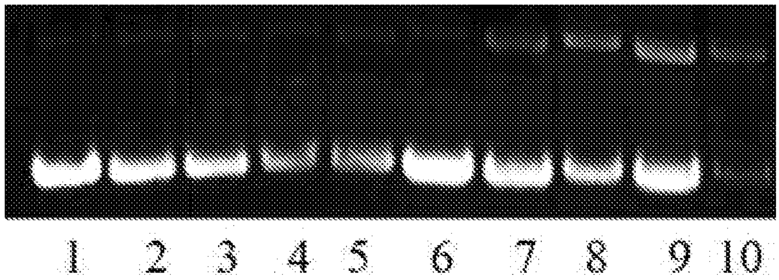


Figure 3

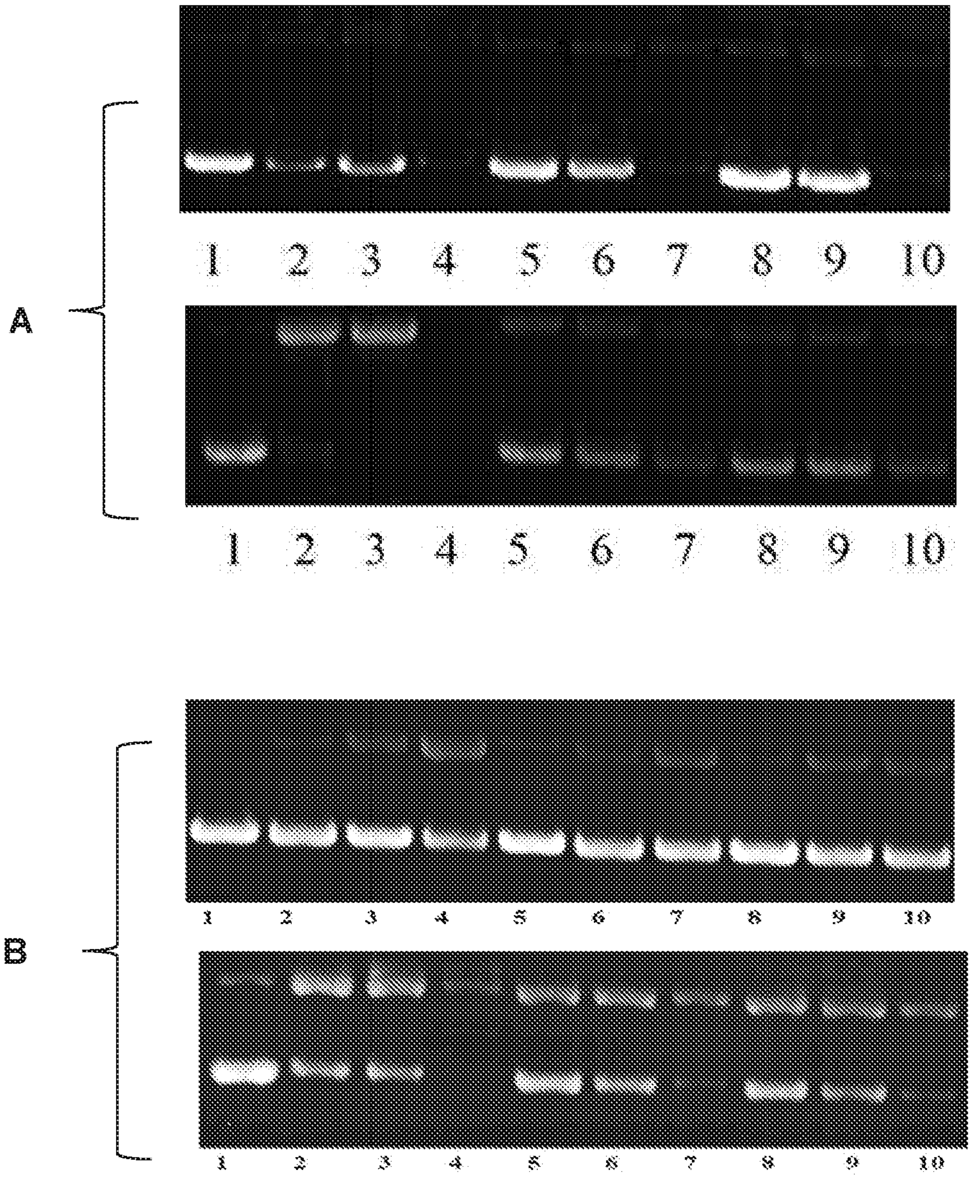


Figure 4

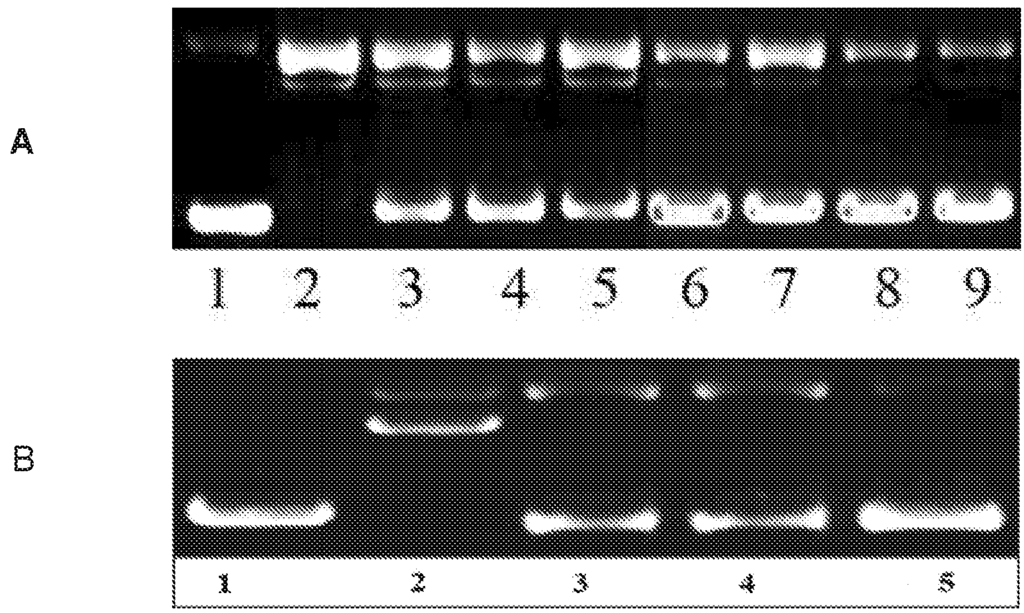


Figure 5

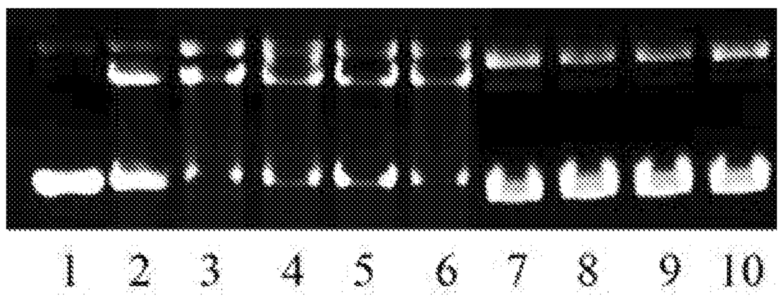


Figure 6

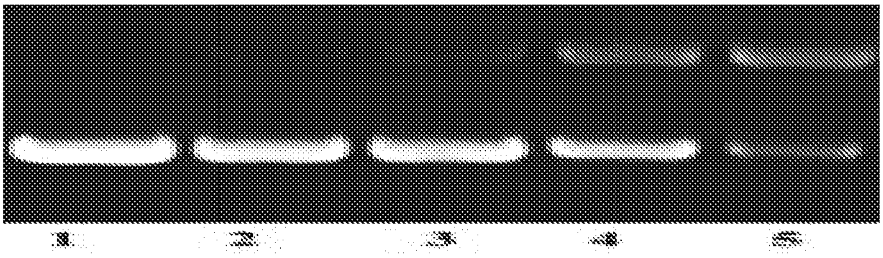


Figure 7

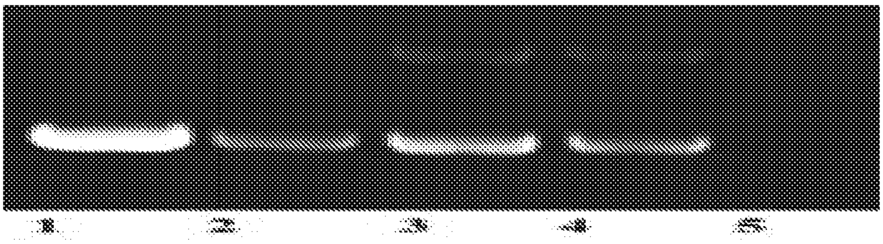


Figure 8

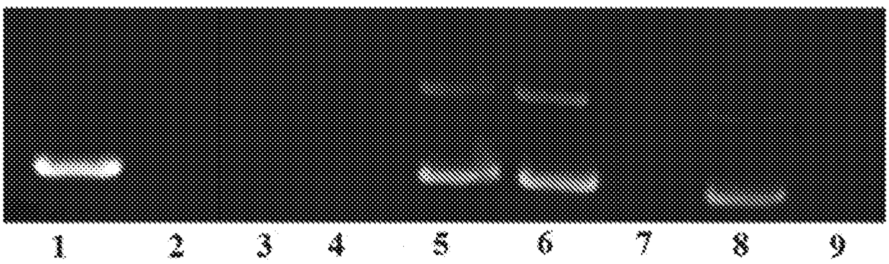


Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102327231
- CN 1861603 A
- US 9890181 B2
- WO 2018086242 A1
- US 2013237503 A1
- WO 2017000379 A1
- CN 105669735 A
- CN 107474064 A